# FASCICULE DE BREVET EUROPEEN

(11) **EP 3 049 382 B1**
(45) Date de publication et mention de la délivrance du brevet: **09.05.2018**
(21) Numéro de dépôt: 14784307.2
(22) Date de dépôt: 17.09.2014
(51) Int. Cl.: C07C 17/20, C07C 21/18, C07C 17/25

(54) **PROCEDE DE FLUORATION EN PHASE GAZ**
VERFAHREN ZUR FLUORIERUNG IN DER GASPHASE
METHOD OF FLUORINATION IN THE GASEOUS PHASE

(30) Priorité: 24.09.2013 FR 1359143
(43) Date de publication de la demande: 03.08.2016
(73) Titulaire: Arkema France, 92700 Colombes (FR)
(72) Inventeur: WENDLINGER, Laurent, 69510 Soucieu En Jarrest (FR)
(74) Mandataire: Leca, François Michel
(86) Numéro de dépôt international: PCT/FR2014/052309
(87) Numéro de publication internationale: WO 2015/044558

(56) Documents cités:
- WO-A1-2008/054781
- WO-A1-2009/158321
- WO-A1-2012/098420
- WO-A2-2008/060616

## Description

La présente invention concerne un procédé de fluoration en phase gaz en présence d'un catalyseur.

Le 2,3,3,3-tetrafluoropropène (HFO-1234yf), du fait de son faible pouvoir de réchauffement Global Warming Potential, est considéré comme un candidat potentiel au remplacement du HFC-134a dans la climatisation automobile.

Le 2,3,3,3-tetrafluoropropène peut être obtenu à partir du 1,2,3,3,3-pentafluoropropène (HFO-1225ye) en faisant réagir le HFO-1225ye avec de l'hydrogène en présence d'un catalyseur d'hydrogénation pour donner du 1,1,1,2,3-pentafluoropropane (HFC-245eb) ; le HFC-245eb ainsi formé est ensuite soumis à une étape de déhydrofluoration en présence de l'hydroxyde de potassium (Knunyants et al., Journal of Academy of Sciences of the USSR, page 1312-1317, August, 1960).

Le 2,3,3,3-tetrafluoropropène peut également être obtenu en faisant réagir le 2-chloro,3,3,3-trifluoropropène (HCFO-1233xf) avec de l'HF en présence d'un catalyseur pour donner dans un premier temps le 2-chloro-2,3,3,3-tetrafluoropropane (HCFC-244bb), puis le HCFC-244bb est dehydrochloré sur un deuxième catalyseur (WO 2007/079431).

Le 2,3,3,3-tetrafluoropropène peut également être obtenu à partir des pentachloropropanes ou tetrachloropropènes en passant par le 2-chloro,3,3,3-trifluoropropène comme intermédiaire.

Certains oxydes métalliques sont utilisés comme catalyseur ou précurseur catalytique dans la fabrication des hydrofluorocarbures saturés et insaturés. Ainsi, l'oxyde de chrome, en particulier le chrome (III) en présence d'HF à température élevée donne un mélange de fluorure de chrome et d'oxyfluorure de chrome, très actif dans les réactions de substitution d'au moins un atome de chlore (C-Cl) par un atome de fluor (C-F). Cette substitution est une étape clé dans la majorité des procédés de fluoration des hydrochlorocarbures.

Les catalyseurs à base d'oxyde de chrome dans les réactions de fluoration, en phase gaz, peuvent être préparés par réduction des trioxydes de chrome (VI) ou par précipitation d'un sel de chrome (III) avec des bases.

Outre l'oxyde de chrome et/ou l'oxyfluorure de chrome, les catalyseurs de fluoration peuvent renfermer au moins un autre métal tel que le zinc, le nickel, le magnésium et le cobalt.

Les catalyseurs de fluoration peuvent également être supportés.

Le document WO 2010/123154 décrit un procédé de fabrication du HFO-1234yf en faisant réagir le HCFO-1233xf avec de l'HF en présence d'oxygène à l'aide d'un catalyseur d'oxyde de chrome de formule CrOₘ, avec 1,5 < m < 3, éventuellement fluoré. Ce document enseigne que pour obtenir une bonne sélectivité en HFO-1234yf, la température de réaction doit être comprise entre 330 et 380°C à une pression de 0,08 à 0,2 MPa avec un ratio molaire d'oxygène par rapport au HCFO-1233xf compris entre 0,1 et 1 et un ratio molaire d'HF par rapport au HCFO-1233xf compris entre 4 et 30.

Le document WO 2010/123154 ne s'intéresse qu'à la sélectivité du HFO-1234yf pour une durée de réaction très faible (maximum 45 heures). Ainsi, la conversion n'est que de 6,2 % à l'exemple 3 après 45 heures de réaction.

Le document WO 2012/098420 décrit la préparation du 2,3,3,3-tétrafluoropropène à partir de 1,1,1,2,3-pentachloropropane en phase gazeuse en présence d'un catalyseur mixte Cr-Ni. Le document WO 2009/158321 décrit la préparation du 2,3,3,3-tétrafluoropropène à partir de 1,1,1,2-tétrachloropropène en phase gazeuse en présence d'un catalyseur d'oxyde de chrome. Le document WO 2008/054781 décrit un procédé de production d'au moins un composé sélectionné parmi le groupe consistant en 1,1,1,2,2-pentafluoropropane, 2,3,3,3-tétrafluoropropène et 2-chloro-3,3,3-trifluoropropène en phase gazeuse en présence d'un catalyseur d'oxyfluorure de chrome et de cobalt. Le document WO 2008/060616 décrit la production du 1,2,3,3,3-pentafluoropropène à partir de 1-chloro-2,3,3,3-tétrafluoropropène en phase gazeuse en présence d'un catalyseur d'oxyfluorure de chrome comprenant du zinc.

Or, pour qu'un procédé soit viable industriellement non seulement la sélectivité doit être élevée mais aussi la conversion. En outre, les performances doivent être quasi-constantes dans le temps.

La demanderesse a maintenant mis au point un procédé de fabrication d'au moins un composé de formule (II) : CF₃-CX(Z)ₙ-CHX(Z)ₙ dans laquelle X représente indépendamment un atome d'hydrogène, de fluor ou de chlore, Z représente indépendamment un atome d'hydrogène ou de fluor et n = 0 ou 1, pouvant être mis en oeuvre industriellement et ne présentant pas les inconvénients de l'art antérieur. Plus précisément, la présente invention fournit un procédé de fabrication d'au moins un composé de formule (II) à partir d'au moins un composé de formule (I) : CX(Y)₂-CX(Y)ₘ-CHₘXY dans laquelle X et Y représentent indépendamment un atome d'hydrogène, de fluor ou de chlore et m = 0 ou 1.
Le procédé selon la présente invention comprend au moins une étape au cours de laquelle au moins un composé de formule (I) réagit ou réagissent avec de l'HF en phase gazeuse en présence d'un catalyseur de fluoration pour donner au moins un composé de formule (II) caractérisé en ce que le catalyseur est à base d'oxyfluorure de chrome renfermant comme co-metal le nickel et au moins un métal de terre rare.

Comme composés de formule (II), on peut citer notamment le 2,3,3,3-tetrafluoropropène (HFO-1234yf), le 1,3,3,3-tetrafluoropropène (HFO-1234ze), le 1-chloro- 3,3,3-trifluoro-1-propène (HCFO-1233zd), le 2-chloro-3,3,3-trifluoro-1-propène (HCFO-1233xf), le 1,2-dichloro-3,3,3-trifluoropropane (HCFC-243db), le 2-chloro-2,3,3,3-tetrafluoropropane (HCFC-244bb), le 1,1,1,2,2-pentafluoropropane (HFC-245cb), le 1-chloro-1,3,3,3-tetrafluoropropane (HCFC-244fa), le 1,1,1,3,3-pentafluoropropane (HFC-245fa).

De préférence, les composés de formule (II) sont choisis parmi le 2,3,3,3-tetrafluoropropène (HFO-1234yf), le 1,3,3,3-tetrafluoropropène (HFO-1234ze), le 1-chloro-3,3,3-trifluoro-1-propène (HCFO-1233zd).
Comme composés de formule (I), on peut citer notamment les tetrachloropropènes, en particulier le 1,1,2,3-tetrachloropropène (HCO-1230xa), le 2,3,3,3,-tetrachloropropène (HCO-1230xf), le 1,1,3,3-tetrachloropropène (HCO-1230za) et le 1,3,3,3-tetrachloropropène (HCO-1230zd), les pentachloropropanes, en particulier le 1,1,1,2,3-pentachloropropane (HCC-240db), le 1,1,1,3,3-pentachloropropane (HCC-240fa) et le 1,1,2,2,3-pentachloropropane (HCC-240aa).

Selon un mode de réalisation de la présente invention, le 2,3,3,3-tetrafluoropropène est fabriqué à partir des halopropanes de formules CX₃CHClCH₂X et CX₃CFXCH₃, halopropènes de formules CX₃CCNCH₂, CClX₂CCl=CH₂ et CX₂=CClCH₂X, avec X représentant indépendamment un atome de fluor ou de chlore.

De préférence, le 2,3,3,3-tetrafluoropropène est fabriqué à partir du 1,1,1,2,3-pentachloropropane, du 1,1,2,3,tetrachloropropène, du 1,2-dichloro-3,3,3-trifluoropropane, du 1,1,1,2,2-pentafluoropropane et/ou du 2-chloro-3,3,3-trifluoro-1-propène.

Selon ce mode, le 2,3,3,3-tetrafluoropropène est avantageusement fabriqué à partir du 1,1,1,2,3-pentachloropropane et/ou du 2-chloro- 3,3,3-trifluoro-1-propène.

Selon un autre mode de réalisation de la présente invention, le 1,3,3,3-tetrafluoropropène est fabriqué à partir du 1,1,1,3,3-pentachloropropane, du 1,1,3,3-tetrachloropropène, du 1,1-dichloro-3,3,3-trifluoropropane, du 1,1,1,3,3-pentafluoropropane et/ou du 1-chloro-3,3,3-trifluoro-1-propène.
Selon ce mode, le 1,3,3,3-tetrafluoropropène est de préférence fabriqué à partir du 1,1,1,3,3-pentachloropropane et/ou du 1-chloro-3,3,3-trifluoro-1-propène.

Selon un autre mode de réalisation de la présente invention, le 1-chloro-3,3,3-trifluoro-1-propène en particulier l'isomère trans est fabriqué à partir du 1,1,1,3,3-pentachloropropane et/ou du 1,1,3,3-tetrachloropropène.

Comme métal de terre rare entrant dans la composition du catalyseur utilisé dans le procédé selon la présente invention, on peut citer notamment le lanthane, le cérium, le praséodyme, le néodyme, le prométhium, le samarium, l'europium, le gadolium, le terbium, le dysprosium, le holmium, l'erbium, le thulium, l'ytterbium, le lutécium, l'yttrium et le scandium.

Les métaux de terre rares recyclés peuvent convenir dans la préparation du catalyseur.

Les métaux de terre rare préférés sont le cérium, le lanthane et le praséodyme.

Le rapport atomique de métal ou métaux terre rare/chrome dans le catalyseur est de préférence compris entre 0,001-0,1 et avantageusement compris entre 0,001-0,02.

Le rapport atomique du co-métal nickel/chrome dans le catalyseur est de préférence compris entre 0,5 et 5, et avantageusement compris entre 0,7 et 2.

Quelque soit le mode de réalisation de l'invention la température de l'étape de fluoration peut être comprise entre 100 et 500°C, de préférence comprise entre 180 et 420°C et avantageusement comprise entre 280 et 420°C. Le ratio molaire HF par rapport à la totalité des composés de formule (I) à réagir est de préférence compris entre 5 et 40 et avantageusement compris entre 10 et 25.

Quel que soit le mode de réalisation, l'étape de fluoration peut être mise en oeuvre en présence d'un agent d'oxydant avec un ratio molaire d'agent d'oxydant par rapport au(x) composé(s) de formule (I) de préférence compris entre 0,005 et 2.

L'étape de fluoration est en général mise en oeuvre à une pression comprise entre 0,1 et 20 bar, de préférence entre 1 et 7 bar.

L'agent oxydant peut être choisi parmi l'oxygène, le chlore et l'air.

Le catalyseur utilisé peut être massique ou supporté.

Comme support de catalyseur, on peut citer notamment le carbone ou les dérivés de magnésium notamment des halogénures tel que MgF₂ ou des oxyhalogénures de magnésium tel que les oxyfluorures ou l'alumine, éventuellement activée ou les dérivés d'aluminium notamment des halogénures, tel que AlF₃ ou oxyhalogénures d'aluminium tel que oxyfluorure.

Le catalyseur utilisé dans la présente invention peut être préparé par coprécipitation des sels à base de chrome, de nickel et de métaux de terre rares correspondants éventuellement en présence d'un support.

Le catalyseur peut également être préparé par cobroyage des oxydes correspondants. Préalablement à la réaction de fluoration le catalyseur est soumis à une étape d'activation par de l'HF.

La température du traitement avec de l'HF peut être comprise entre 100 et 450°C, de préférence entre 200 et 300°C pour une durée comprise entre 1 et 50 heures.

Selon un autre mode de réalisation, l'activation des catalyseurs peut être mise en oeuvre en au moins une étape avec un traitement au mélange d'HF et d'agent oxydant. L'agent oxydant peut représenter entre 2 et 98 % molaire par rapport au mélange d'HF et agent oxydant et la température d'activation peut varier entre 200 et 450°C pour une durée comprise entre 10 et 200 heures.

Les étapes d'activation peuvent être mises en oeuvre à pression atmosphérique ou sous pression jusqu'à 20 bar.
Le support peut être préparé à partir d'alumine à porosité élevée. Dans une première étape l'alumine est transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200°C et 450°C, de préférence entre 250°C et 400°C). Le support est ensuite imprégné à l'aide de solutions aqueuses de sels de chrome, de nickel et de métal de terre rare, ou à l'aide de solutions aqueuses d'acide chromique, de sel de nickel, et de sels ou d'oxydes de terre rares et de méthanol (servant de réducteur au chrome). Comme sels de chrome, de nickel et de métaux de terre rare, on peut employer des chlorures, ou d'autres sels tels que, par exemple, les oxalates, formiates, acétates, nitrates et sulfates ou le bichromate de nickel, et de métaux de terre rare, pour autant que ces sels soient solubles dans la quantité d'eau susceptible d'être absorbée par le support.

Le catalyseur peut aussi être préparé par imprégnation directe de l'alumine (qui en général est activée) à l'aide des solutions des composés de chrome, de nickel, et de métaux de terre rare, ci-dessus mentionnées. Dans ce cas, la transformation d'au moins une partie (par exemple 70% ou plus) de l'alumine en fluorure d'aluminium ou oxyfluorure d'aluminium s'effectue lors de l'étape d'activation du métal du catalyseur.

Les alumines activées susceptibles d'être utilisées pour la préparation du catalyseur sont des produits bien connus, disponibles dans le commerce. Elles sont généralement préparées par calcination d'hydrates d'alumine (hydroxydes d'aluminium) à une température comprise entre 300°C et 800°C. Les alumines (activées ou non) peuvent contenir des teneurs importantes (jusqu'à 1000 ppm) de sodium sans que cela nuise aux performances catalytiques.

De préférence, le catalyseur est conditionnée ou activé, c'est-à-dire transformé en constituants actifs et stables (aux conditions réactionnelles) par une opération préalable dite d'activation. Ce traitement peut être réalisé soit "in situ" (dans le réacteur de fluoration) ou bien dans un appareillage adéquat conçu pour résister aux conditions d'activation.

Après imprégnation du support, le catalyseur est séché à une température comprise entre 100°C et 350 °C, de préférence 220°C à 280°C en présence d'air ou d'azote.

Le catalyseur séché est ensuite activé en une ou deux étapes avec de l'acide fluorhydrique, éventuellement en présence d'un agent oxydant. La durée de cette étape d'activation par fluoration peut être comprise entre 6 et 100 heures et la température comprise entre 300 et 400°C.
Le procédé selon la présente invention peut être mis en oeuvre en continu ou discontinu.

La présente invention fournit en outre un procédé d'isomérisation ou de déhydrohalogénation en présence du catalyseur tel que décrit ci-dessus.

A titre d'exemple d'isomérisation en peut citer notamment l'obtention de 2,3,3,3-tetrafluoropropène à partir de 1,3,3,3-tetrafluoropropène et la réaction inverse, l'obtention de 2-chloro-3,3,3-trifluoro-1-propène à partir de 1-chloro-3,3,3-trifluoro-1-propène et la réaction inverse, l'obtention de l'isomère trans du 1-chloro-3,3,3-trifluoro-1-propène à partir de l'isomère cis et l'obtention de l'isomère trans du 1,3,3,3-tetrafluoropropène à partir de l'isomère cis.

A titre d'exemple de déhydrohalogénation, on peut citer notamment l'obtention de 2,3,3,3-tetrafluoropropène à partir de 2-chloro-2,3,3,3-tetrafluoropropane et/ou de 1,1,1,2,2-pentafluoropropane et l'obtention de 1,3,3,3-tetrafluoropropène à partir de 1-chloro-1,3,3,3-tetrafluoropropane et/ou de 1,1,1,3,3-pentafluoropropane.

Comme composé de formule (I), on peut citer également le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoroprapane.

### PARTIE EXPERIMENTALE

L'appareillage utilisé comprend un réacteur tubulaire en INCONEL® 600 d'un diamètre interne de 19 mm, chauffé par un four tubulaire. Le réacteur est équipé de contrôleurs de pression et de température. Les réactifs sont préchauffés et mélangés, grâce à un mélangeur statique, puis introduits en phase gazeuse dans la partie supérieure du réacteur.

A la sortie du réacteur, on prélève un échantillon des produits de la réaction qui est analysé en ligne par Chromatographie en phase gazeuse. L'analyse est réalisée en utilisant une colonne CP Sil 8CB, dimensions 50 m x 0,32 mm x 5 µm et une colonne garnie 1% SP1000 / Carbopack B, 60/80 mesh de 5m de longueur. Les programmations de température des fours sont les suivantes : 40°C pendant 10 min puis pente de 10°C / min jusqu'à 250°C et 40°C pendant 20 min puis pente de 10°C / min jusqu'à 180°C.

Le temps de contact est défini comme le rapport du volume du lit de catalyseur sur le débit volumique total dans les conditions expérimentales de température et de pression. Le rapport molaire HF est défini comme le rapport entre le débit molaire d'HF et le débit molaire de HCFO-1233xf. La réaction est effectuée en présence d'air. Le rapport molaire d'oxygène est défini comme le rapport entre le débit molaire d'oxygène et le débit molaire de HCFO-1233xf.

### Exemple 1 (comparatif) : fluoration de HCFO-1233xf

Le catalyseur utilisé est un catalyseur Ni-Cr/AlF₃ préparé comme suit.

Dans un évaporateur rotatif, on place 343 g d'un support obtenu dans une étape précédente par fluoration d'alumine GRACE HSA en lit fixe vers 280°C à l'aide d'air et d'acide fluorhydrique (concentration volumique de 5 à 10% de cet acide dans l'air). L'alumine GRACE HSA de départ présente les caractéristiques physicochimiques suivantes : billes de 0,5-2 mm de diamètre surface BET = 220 m²/g, volume poreux = 1,3 cm³/g.

On prépare par ailleurs deux solutions aqueuses séparées :
(a) solution chromique additionnée de chlorure de nickel contenant :
   - anhydride chromique CrO₃ = 55 g
   - chlorure de nickel hexahydraté NiCl₂.6H₂O = 130 g
   - eau = 63 g
(b) Solution méthanolique contenant :
   - méthanol = 81 g
   - eau = 8 g

Ces deux solutions sont introduites simultanément à une température de 40°C sous pression atmosphérique et en 2 heures environ, sur le support en agitation. Après une étape de maturation sous azote, le catalyseur est séché sous azote, puis sous vide à 65°C puis vers 90°C pendant 6 heures.

La fluoration de HCFO-1233xf est réalisée dans le réacteur décrit ci-dessus en chargeant 73 cm³ de catalyseur Ni-Cr supporté sur AlF₃.

Après chargement, le solide est traité à une température comprise entre 320°C et 390°C en présence d'un mélange d'acide fluorhydrique et d'azote (concentration volumique de 5 à 10% de cet acide dans de l'azote).

Le processus d'activation comprend ensuite 5 cycles de :
- fluoration du catalyseur en effectuant la réaction de fluoration pendant 6 à 30 heures dans les conditions énoncées ci-dessous,
- traitement sous air à 370°C et 1,5 L/h pendant 64 heures.

La réaction est effectuée en alimentant en continu 3,4 g/h d'HF anhydre et 1,0 g/h de HCFO-1233xf à pression atmosphérique et à une température de 350°C. Ainsi, le temps de contact est de 29 s, le rapport molaire HF est de 22. La quantité d'oxygène est de 8% en moles par rapport à la quantité de HCFO-1233xf.

Les résultats sont présentés dans le tableau ci-dessous.

### Exemple 2 : fluoration de HCFO-1233xf

Le catalyseur utilisé est un catalyseur Ni-Cr-La/AlF₃ préparé comme précédemment, la solution chromique additionnée de chlorure de nickel et de chlorure de lanthane contenant :
- anhydride chromique CrO₃ = 55 g
- chlorure de nickel hexahydraté NiCl₂.6H₂O = 130 g
- chlorure de lanthane hexahydraté LaCl₃.6H₂O = 2 g
- eau = 63 g

La fluoration de HCFO-1233xf est réalisée dans le réacteur décrit ci-dessus en chargeant 73 cm³ de catalyseur Ni-Cr-La supporté sur AlF₃.
Le processus d'activation est identique et la réaction est également effectuée en alimentant en continu 3,4 g/h d'HF anhydre et 1,0 g/h de HCFO 1233xf à pression atmosphérique et à une température de 350°C. La quantité d'oxygène est de 8% en moles par rapport à la quantité de HCFO-1233xf.

Les résultats complets sont présentés dans le tableau ci-dessous.

| | Durée de réaction | Conversion (%) | Sélectivité en HFO-1234yf | Sélectivité en HFC-245cb | Sélectivité autres |
|---|---|---|---|---|---|
| Exemple 1 (comparatif) | 7 h | 69,7 % | 65,7 % | 32,8 % | 1,5 % |
| | 33 h | 54,7 % | 64,6 % | 32,3 % | 3,1 % |
| Exemple 2 | 8 h | 70,2 % | 66,4 % | 32,4 % | 1,2 % |
| | 41 h | 63,8 % | 65,1 % | 32,6 % | 2,3 % |

## Revendications

1. Procédé de fabrication d'au moins un composé de formule (II) : CF₃-CX(Z)ₙ-CHX(Z)ₙ dans laquelle X représente indépendamment un atome d'hydrogène, de fluor ou de chlore, Z représente indépendamment un atome d'hydrogène ou de fluor et n = 0 ou 1, comprenant au moins une étape au cours de laquelle au moins un composé de formule (I) : CX(Y)₂-CX(Y)ₘ-CHₘXY dans laquelle X et Y représentent indépendamment un atome d'hydrogène, de fluor ou de chlore et m = 0 ou 1, réagit ou réagissent avec de l'HF en phase gazeuse en présence d'un catalyseur de fluoration **caractérisé en ce que** le catalyseur est à base d'oxyfluorure de chrome renfermant au moins le nickel comme co-metal et au moins un métal de terre rare.

2. Procédé selon la revendication 1 **caractérisé en ce que** les composés de formule (II) sont choisis parmi le 2,3,3,3-tetrafluoropropène, le 1,3,3,3-tetrafluoropropène et le 1-chloro-3,3,3-trifluoro-1-propène.

3. Procédé selon la revendication 1 ou 2 **caractérisé en ce que** les composés de formule (I) sont choisis parmi le 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane.

4. Procédé selon l'une quelconque des revendications précédentes **caractérisé en ce que** le 2,3,3,3-tetrafluoropropène est fabriqué à partir des halopropanes de formules CX₃CHClCH₂X et CX₃CFXCH₃, halopropènes de formules CX₃CCNCH₂, CClX₂CCl=CH₂ et CX₂=CClCH₂X, avec X représentant indépendamment un atome de fluor ou de chlore.

5. Procédé selon l'une quelconque des revendications précédentes **caractérisé en ce que** le 2,3,3,3-tetrafluoropropène est fabriqué à partir du 1,1,1,2,3-pentachloropropane, 1,1,2,3,tetrachloropropène, du 1,1,1,2,2-pentafluoropropane et/ou du 2-chloro-3,3,3-trifluoro-1-propène.

6. Procédé selon l'une quelconque des revendications 1 à 3 **caractérisé en ce que** le 1,3,3,3-tetrafluoropropène est fabriqué à partir 1,1,1,3,3-pentachloropropane, 1,1,3,3-tetrachloropropène, du 1,1,1,3,3-pentafluoropropane et/ou du 1-chloro-3,3,3-trifluoro-1-propène.

7. Procédé selon l'une quelconque des revendications 1 à 3 **caractérisé en ce que** le 1-chloro- 3,3,3-trifluoro-1-propène est fabriqué à partir 1,1,1,3,3-pentachloropropane et/ou du 1,1,3,3-tetrachloropropène.

8. Procédé selon l'une quelconque des revendications précédentes **caractérisé en ce que** le ou les métaux de terre rare est ou sont choisi(s) parmi le cérium, le lanthane et le praséodyme.

9. Procédé selon l'une quelconque des revendications précédentes **caractérisé en ce que** le rapport atomique de métal ou métaux de terre rare/chrome dans le catalyseur est compris entre 0,001-0,1, de préférence compris entre 0,001-0,02.

10. Procédé selon l'une quelconque des revendications précédentes **caractérisé en ce que** le rapport atomique du co-métal nickel/chrome dans le catalyseur est compris entre 0,5 et 5 et de préférence compris entre 0,7 et 2.

11. Procédé selon l'une quelconque des revendications précédentes **caractérisé en ce que** le catalyseur est supporté.

12. Procédé selon la revendication 11 **caractérisé en ce que** le support est choisi parmi le carbone, les dérivés de magnésium, l'alumine ou les dérivés d'aluminium.

13. Procédé selon l'une quelconque des revendications précédentes **caractérisé en ce que** le ratio molaire d'HF par rapport aux composés de formule (I) à réagir est compris entre 5 et 40, de préférence entre 10 et 25.

14. Procédé selon l'une quelconque des revendications précédentes **caractérisé en ce que** la température de fluoration est comprise entre 180 et 420°C, de préférence entre 280 et 420°C.

15. Procédé selon l'une quelconque des revendications précédentes **caractérisé en ce que** le catalyseur est soumis à au moins une étape d'activation à l'aide d'un flux comprenant de l'HF.

## Patentansprüche

1. Verfahren zur Herstellung mindestens einer Verbindung der Formel (II) : CF₃-CX(Z)ₙ-CHX(Z)ₙ, wobei X unabhängig ein Wasserstoff-, Fluor- oder Chloratom darstellt, Z unabhängig ein Wasserstoff- oder Fluoratom darstellt, und n = 0 oder 1, umfassend mindestens einen Schritt, in dem mindestens eine Verbindung der Formel (I): CX(Y)₂-CX(Y)ₘ-CHₘXY, wobei X und Y unabhängig ein Wasserstoff-, Fluor- oder Chloratom darstellen, und m = 0 oder 1, mit HF in der Gasphase in Anwesenheit eines Fluorierungskatalysators reagiert oder reagieren, **dadurch gekennzeichnet, dass** der Katalysator auf Chromoxyfluorid, umfassend mindestens Nickel als Co-Metall und mindestens ein Seltenerdmetall, basiert.

2. Verfahren nach Anspruch 1, **dadurch gekennzeichnet, dass** die Verbindungen der Formel (II) ausgewählt werden aus 2,3,3,3-Tetrafluorpropen, 1,3,3,3-Tetrafluorpropen und 1-Chlor-3,3,3-trifluor-1-propen.

3. Verfahren nach Anspruch 1 oder 2, **dadurch gekennzeichnet, dass** die Verbindungen der Formel (I) ausgewählt werden aus 1,1,2,3-Tetrachlorpropen, 2,3,3,3-Tetrachlorpropen, 1,1,3,3-Tetrachlorpropen, 1,3,3,3-Tetrachlorpropen, 1,1,1,2,3-Pentachlorpropan, 1,1,1,3,3-Pentachlorpropan, 1,1,2,2,3-Pentachlorpropan, 1,2-Dichlor-3,3,3-trifluorpropan, 2-Chlor-2,3,3,3-tetrafluorpropan, 1,1,1,2,2-Pentafluorpropan, 1-Chlor-1,3,3,3-tetrafluorpropan und 1,1,1,3,3-Pentafluorpropan.

4. Verfahren nach einem der vorhergehenden Ansprüche, **dadurch gekennzeichnet, dass** 2,3,3,3-Tetrafluorpropen aus Halopropanen der Formeln CX₃CHClCH₂X und CX₃CFXCH₃, Halopropenen der Formeln CX₃CCl=CH₂, CClX₂CCl=CH₂ und CX₂=CClCH₂X hergestellt wird, wobei X unabhängig ein Fluor- oder Chloratom darstellt.

5. Verfahren nach einem der vorhergehenden Ansprüche, **dadurch gekennzeichnet, dass** 2,3,3,3-Tetrafluorpropen aus 1,1,1,2,3-Pentachlorpropan, 1,1,2,3-Tetrachlorpropen, 1,1,1,2,2-Pentafluorpropan und/oder 2-Chlor-3,3,3-trifluor-1-propen hergestellt wird.

6. Verfahren nach einem der Ansprüche 1 bis 3, **dadurch gekennzeichnet, dass** 1,3,3,3-Tetrafluorpropen aus 1,1,1,3,3-Pentachlorpropan, 1,1,3,3-Tetrachlorpropen, 1,1,1,3,3-Pentafluorpropan und/oder 1-Chlor-3,3,3-trifluor-1-propen hergestellt wird.

7. Verfahren nach einem der Ansprüche 1 bis 3, **dadurch gekennzeichnet, dass** 1-Chlor-3,3,3-trifluor-1-propen aus 1,1,1,3,3-Pentachlorpropan und/oder 1,1,3,3-Tetrachlorpropen hergestellt wird.

8. Verfahren nach einem der vorhergehenden Ansprüche, **dadurch gekennzeichnet, dass** das oder die Seltenerdmetalle aus Cer, Lanthan und Praseodym ausgewählt werden.

9. Verfahren nach einem der vorhergehenden Ansprüche, **dadurch gekennzeichnet, dass** das Atomverhältnis des Seltenerdmetalls oder der Seltenerdmetalle/Chrom im Katalysator zwischen 0,001-0,1, vorzugsweise zwischen 0,001-0,02, beträgt.

10. Verfahren nach einem der vorhergehenden Ansprüche, **dadurch gekennzeichnet, dass** das Atomverhältnis des Co-Metalls Nickel/Chrom im Katalysator zwischen 0,5 und 5 und vorzugsweise zwischen 0,7 und 2 beträgt.

11. Verfahren nach einem der vorhergehenden Ansprüche, **dadurch gekennzeichnet, dass** der Katalysator geträgert ist.

12. Verfahren nach Anspruch 11, **dadurch gekennzeichnet, dass** der Träger ausgewählt wird aus Kohlenstoff, Derivaten von Magnesium, Aluminiumoxid oder Derivaten von Aluminium.

13. Verfahren nach einem der vorhergehenden Ansprüche, **dadurch gekennzeichnet, dass** das Molverhältnis von HF im Verhältnis zu den umzusetzenden Verbindungen der Formel (I) zwischen 5 und 40, vorzugsweise zwischen 10 und 25, beträgt.

14. Verfahren nach einem der vorhergehenden Ansprüche, **dadurch gekennzeichnet, dass** die Fluorierungstemperatur zwischen 180 und 420°C, vorzugsweise zwischen 280 und 420°C, beträgt.

15. Verfahren nach einem der vorhergehenden Ansprüche, **dadurch gekennzeichnet, dass** der Katalysator mindestens einem Aktivierungsschritt mit Hilfe eines HF umfassenden Stroms unterzogen wird.

## Claims

1. Process for manufacturing at least one compound of formula (II): CF₃-CX(Z)ₙ-CHX(Z)ₙ in which X independently represents a hydrogen, fluorine or chlorine atom, Z independently represents a hydrogen or fluorine atom and n = 0 or 1, comprising at least one step during which at least one compound of formula (I): CX(Y)₂-CX(Y)ₘ-CHₘXY in which X and Y independently represent a hydrogen, fluorine or chlorine atom and m = 0 or 1, reacts with HF in the gaseous phase in the presence of a fluorination catalyst, **characterized in that** the catalyst is based on chromium oxyfluoride containing at least nickel as cometal and at least one rare-earth metal.

2. Process according to Claim 1, **characterized in that** the compounds of formula (II) are chosen from 2,3,3,3-tetrafluoropropene, 1,3,3,3-tetrafluoropropene and 1-chloro-3,3,3-trifluoro-1-propene.

3. Process according to Claim 1 or 2, **characterized in that** the compounds of formula (I) are chosen from 1,1,2,3-tetrachloropropene, 2,3,3,3-tetrachloropropene, 1,1,3,3-tetrachloropropene, 1,3,3,3-tetrachloropropene, 1,1,1,2,3-pentachloropropane, 1,1,1,3,3-pentachloropropane, 1,1,2,2,3-pentachloropropane, 1,2-dichloro-3,3,3-trifluoropropane, 2-chloro-2,3,3,3-tetrafluoropropane, 1,1,1,2,2-pentafluoropropane, 1-chloro-1,3,3,3-tetrafluoropropane and 1,1,1,3,3-pentafluoropropane.

4. Process according to any one of the preceding claims, **characterized in that** 2,3,3,3-tetrafluoropropene is manufactured from halopropanes of formulae CX₃CHClCH₂X and CX₃CFXCH₃, halopropenes of formulae CX₃CCl=CH₂, CClX₂CCl=CH₂ and CX₂=CClCH₂X, with X independently representing a fluorine or chlorine atom.

5. Process according to any one of the preceding claims, **characterized in that** 2,3,3,3-tetrafluoropropene is manufactured from 1,1,1,2,3-pentachloropropane, 1,1,2,3-tetrachloropropene, 1,1,1,2,2-pentafluoropropane and/or 2-chloro-3,3,3-trifluoro-1-propene.

6. Process according to any one of Claims 1 to 3, **characterized in that** 1,3,3,3-tetrafluoropropene is manufactured from 1,1,1,3,3-pentachloropropane, 1,1,3,3-tetrachloropropene, 1,1,1,3,3-pentafluoropropane and/or 1-chloro-3,3,3-trifluoro-1-propene.

7. Process according to any one of Claims 1 to 3, **characterized in that** 1-chloro-3,3,3-trifluoro-1-propene is manufactured from 1,1,1,3,3-pentachloropropane and/or 1,1,3,3-tetrachloropropene.

8. Process according to any one of the preceding claims, **characterized in that** the rare-earth metal(s) are chosen from cerium, lanthanum and praseodymium.

9. Process according to any one of the preceding claims, **characterized in that** the atomic ratio of rare-earth metal(s)/chromium in the catalyst is between 0.001-0.1, preferably between 0.001-0.02.

10. Process according to any one of the preceding claims, **characterized in that** the nickel cometal/chromium atomic ratio in the catalyst is between 0.5 and 5 and preferably between 0.7 and 2.

11. Process according to any one of the preceding claims, **characterized in that** the catalyst is supported.

12. Process according to Claim 11, **characterized in that** the support is chosen from charcoal, magnesium derivatives, alumina or aluminum derivatives.

13. Process according to any one of the preceding claims, **characterized in that** the mole ratio of HF relative to the compounds of formula (I) to be reacted is between 5 and 40, preferably between 10 and 25.

14. Process according to any one of the preceding claims, **characterized in that** the fluorination temperature is between 180 and 420°C, preferably between 280 and 420°C.

15. Process according to any one of the preceding claims, **characterized in that** the catalyst is subjected to at least one activation step using a stream comprising HF.
